# EUROPEAN PATENT APPLICATION

(11) **EP 1 616 965 A1**
(43) Date of publication of application: **18.01.2006**
(21) Application number: 04016774.4
(22) Date of filing: 16.07.2004
(51) Int. Cl.: C12Q 1/527, G01N 33/573

(54) **Methods and assays for detecting guanylate cyclase activity**

(71) Applicant: AXXAM S.r.l., 20145 Milano (IT)
(72) Inventor: Lohmer, Stefan, 20132 Milano (IT); Corazza, Sabrina, 20132 Milano (IT); Liberati, Chiara, 20132 Milano (IT)
(74) Representative: Banfi, Paolo

(57) **Abstract**

The present invention relates to soluble guanylate cyclase (sGC) enzymes. In particular it provides a cell-based assay system which allows to detect with high efficiency and sensitivity the soluble guanylate cyclase enzymatic activity. In a preferred embodiment, the invention provides a method of screening molecules that interact with sGC by stimulating or inhibiting its enzymatic activity and that can be used in the diagnosis or therapy of sGC-mediated dysfunctions or diseases. The invention further provides gene-constructs, vectors, and cells for use in said method.

## Description

The present invention relates to soluble guanylate cyclase (sGC) enzymes. More specifically it provides a cell-based assay system which allows to detect with high efficiency and sensitivity the soluble guanylate cyclase enzymatic activity. In a preferred embodiment, the invention provides a method of screening molecules that interact with sGC by stimulating or inhibiting its enzymatic activity and that can be used in the diagnosis or therapy of sGC-mediated dysfunctions or diseases. The invention further provides gene-constructs, vectors, and cells for use in said method.

### BACKGROUND OF THE INVENTION

Adenylate cyclases and guanylate cyclases synthesize the intracellular second messengers cAMP and cGMP, respectively, in response to a variety of regulatory signals. Mammalian adenylate cyclases are intrinsic plasma membrane proteins that contain a duplicated module consisting of a hexahelical transmembrane region followed by a roughly 40-kDa cytosolic domain (Sunahara, et al. (1996) *Annu. Rev. Pharm. Toxicol.* 36, 461-480). Highly conserved and homologous sequences within the two cytosolic domains contribute to the active site of the enzyme (Tang, et al. (1995) *Science* 268, 1769-1772). The x-ray crystal structure of this soluble domain of the enzyme complexed with both activators and inhibitors has also been solved (Tesmer, et al. (1997) *Science* 278, 1907-1916).

Guanylate cyclases exist as both soluble and membrane-bound species. Both types of the enzyme contain cytoplasmic domains similar to those of the adenylate cyclases (Garbers, D.L., et al. (1994) *Mol. Biol. Cell* 5, 1-5). Membrane-bound guanylate cyclases are monomers stimulated by the natriuretic peptides, whereas the soluble guanylate cyclase (sGC) exists as a heterodimer consisting of an α and a β subunit (both of which are required for catalysis) containing heme as prosthetic group.

Guanylate cyclase (GC) belongs to a family of enzymes which catalyzes the conversion of guanosine 5'- triphosphate (GTP) to cyclic guanosine 3', 5'-monophosphate.(cGMP).

By formation of cGMP as second messenger, sGC plays an important role in a variety of extracellular signals and different physiological processes, i.e. vasodilatation, platelet aggregation and adhesion and neural transmission. The most important physiological activators of sGC are NO and NO-related compounds, which activate the enzyme upon binding to the heme moiety.

Binding of nitric oxide (NO) to a prosthetic heme group causes marked activation of soluble guanylate cyclases. The pathogenesis of various cardiovascular diseases has been linked to an inappropriate activation of sGC. The concept of NO dependent sGC activation as a mechanism of action has been widely validated by the clinical use of NO donors.

Soluble guanylate cyclase is detectable in organs such as, for example, the heart, lung, liver, kidney, and brain of all mammals, including humans. In pathological processes or in processes relevant for pathological events, the oxidation state of the heme group iron in soluble guanylate cyclase may play an essential part. A higher proportion of soluble guanylate cyclase with oxidized heme group iron would result in the possibility of diminishing activation of soluble guanylate cyclase by endogenous NO. This might lead, *inter alia,* to an increase in blood pressure, activation of platelets, increased proliferation of cells or enhanced adhesion of cells, to permanent high blood pressure, stable or unstable angina pectoris, thromboses, myocardial infarct, strokes, pulmonary edemas, erectile dysfunction, uncontrolled tissue growth with tumor formation, diabetes, renal dysfunctions, hepatic dysfunctions, or vascular dysfunctions.

A mutated GC enzyme in which the three residues of the active site of guanylate cyclase are substituted with those of adenylate cyclase has been shown to have a completely changed nucleotide specificity (Sunahara, R.K. et al 1998). The mutated guanylate cyclase enzyme, in particular, has ATP as substrate without having influenced the responsiveness to the respective GC stimulators. This mutated form of soluble guanylate cyclase is useful for the detection of Nitric Oxide induced cAMP production.

Traditional methods for the detection of soluble guanylate cyclase modulators are:
- radioactive methods in which nucleotide precursors are used
- cellular systems based on calcium channels sensitive to cyclic nucleotides
- in-vivo methods in which the effect of stimulators of GC are tested on tissues or in animal models.

### DISCLOSURE OF THE INVENTION

Object of the present invention is to provide a cell-based assay useful for the determination of the enzymatic activity of soluble guanylate cyclase (sGC). A further object of the invention is the provision of a method of screening molecules that interact with sGC, utilizing said cell-based assay.

According to the invention, the sGC enzymatic activity is determined by coupling a sGC enzyme with modified substrate specificity to a cAMP sensitive reporter construct in a suitable eukaryotic or prokaryotic cell, whereby the activity of the reporter gene is indicative of the sGC enzymatic activity. The sGC enzyme with modified substrate specificity is able to catalyze the conversion of ATP into cyclic AMP without affecting the allosteric regulation of the natural enzyme. The sGC substrate specificity can be modified by replacing the amino acid residues involved in nucleotide binding with the corresponding residues present in the active site of adenylate cyclase. For example, when rat sGC is used, the amino acids Arg592 of α subunit, Glu473 and Cys541 of β subunit are respectively changed into Gln, Lys and Asp, to confer ATP substrate-specificity. More details on the modification of sGC substrate specificity can be found in Sunahara et al. (1998), which is hereby incorporated by reference.

The cAMP sensitive reporter construct contains a reporter gene functionally linked to a cAMP-responsive promoter sensitive to intracellular cAMP levels. In a preferred embodiment, the cAMP-responsive promoter is a MRE/CRE (Multiple Response Element - Ray A, et al. 1989; cAMP Responsive Element: Fink, J.S. et al. 1988) inducible promoter containing from 1 to 4 MREs and from 1 to 5 CRE elements. Preferably, the MRE-CRE inducible promoter sensitive to increased intracellular cAMP levels contains 3 MRE and 5 CRE element repetitions.

The MREs correspond to the human Interlukin-6 promoter region which enables the cell line to respond to Gα, Gq and Gs signalling (MRE: Ray A, Sassone-Corsi P, Sehgal PB.,1989; Fitzgerald, LR, Manan, IJ, Dytko, GM, Wu, HL, Nambi,P, 1999). Each MRE element is 32 bp and corresponds to the sequence: ATGCTAAAGGACGTCACATTGCACAATCTTAA.

The CRE sequence ("TGACGTCA") contains one or multiple copies of the conserved sequence motif CGTCA (Fink, J.S. et al. 1988). This element is similar to sequences in other genes known to be regulated by cAMP and to sequences in several viral enhancers.

The reporter construct may further contain transcription-regulating elements, selectable markers and viral promoters.

Suitable reporter genes that may be used according to the invention include, but are not limited to, luciferase, Green Fluorescent Proteins (GFP) and photoprotein genes. This system, however, is very useful not only for reporter gene transcription, but also for the expression and induction of any gene of interest such as the G-protein coupled receptor families (GPCRs), the ion channels and the nuclear hormone receptors.

The invention in addition provides vectors and host cells containing the sGC-encoding gene and/or the reporter gene construct. Preferably, the host cell is a CHO cell line (hereafter, the CHO cell line containing the described MRE- CRE elements driving the expression of a luciferase reporter gene will be referred to as CHO cAMPL). Host cell lines according to the invention are conveniently used as a tool for detection of intracellular NO-release.

In a preferred embodiment, the invention provides a method for determining the sGC enzymatic activity in a cultured eukaryotic or prokaryotic cell, which comprises:
A) introducing into the cell:
   i) a vector encoding a reporter gene functionally linked to a CRE or MRE-CRE inducible promoter, and, simultaneously or separately,
   ii) a vector encoding a sGC with substrate specificity for ATP, i.e able to catalyze the conversion of ATP into cyclic AMP;
B) incubating the cell with a compound that stimulates or inhibits sGC enzymatic activity;
C) determining the activity of the reporter gene.

In a preferred embodiment, the invention is directed to a method of identifying molecules that modulate sGC activity, which comprises:
A) introducing into the cell:
   i) a vector encoding a reporter gene functionally linked to a CRE or MRE-CRE inducible promoter,
      and, simultaneously or separately,
   ii) a vector encoding sGC with substrate specifcity for ATP,
B) incubating the cell with a candidate molecule;
C) determining the activity of the reporter gene.

Molecules that stimulate or inhibit sGC activity can be selected by measuring the reporter gene expression/activity. The assay can be carried out in the presence or absence of known NO-releasing substances which activate the enzyme upon NO-binding to its heme moiety. Both NO-releasing molecules and NO-independent sGC activators (and inhibitors which are not specific for the catalytic site, e.g. ODQ) can be conveniently assayed with the method of the invention. The selected compounds represent valuable candidate for the treatment of diseases related to sGC dysfunctions. In particular, sGC-stimulating compounds can be used in the therapy of diseases involving inappropriate activation of sGC, especially diseases of the cardiovascular system.

Compared to known assays used so far for determining the sGC activity, the method of the invention is more advantageous in that a) it does not require radioactive reagents, b) it has a higher sensitivity, c) it can be easily adapted to a high throughput format and d) it is performed in a native environment.

In a further embodiment, the invention provides a method of modulating the expression of a gene of interest in cultured eukaryotic or prokaryotic cell, which comprises:
A) introducing into the cell:
   i) a vector encoding the target gene functionally linked to a CRE or MRE-CRE inducible promoter;
      and, simultaneously or separately,
   ii) a vector encoding sGC with substrate specificity for ATP,
B) incubating the cell with a molecule able to stimulate sGC in a NO-dependent or NO-independent manner.

A typical assay according to the invention, the conditions and reagents therein utilized, are disclosed below in greater detail.

### EXPERIMENTAL SECTION

Rat wild type sGC α3 and β1 subunits were amplified from brain cDNA by RT-PCR and used as templates for site directed mutagenesis. The amino acids Arg 592 of α subunit, Glu 473 and Cys 541 of β subunit were respectively changed into Gln, Lys and Asp, which confer specificity for adenosine.

An expression vector for the rat mutagenized sGC cDNA was prepared (pIRES from Clontech). A reporter vector was constructed by cloning the MRE-CRE (Multiple Response Element: Ray A, et al. 1989; cAMP Responsive Element: Fink, J.S. et al. 1988) inducible promoter at 5' of the luciferase gene of the pMAMneoluc vector (Clontech).

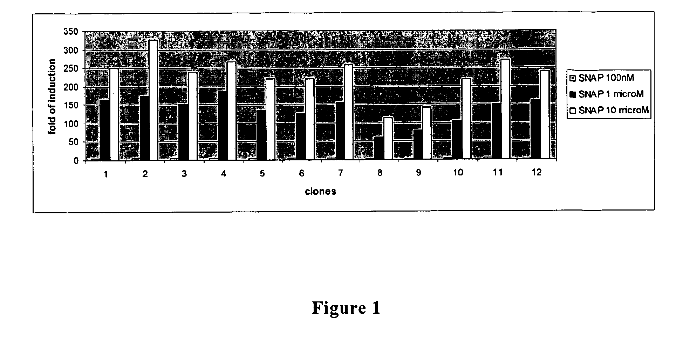

The structure of the MRE-CRE inducible element sensitive to increased intracellular cAMP levels, is the following:
MRE element: 3 (ATGCTAAAGGACGTCACATTGCACAATCTTAA)
CRE element: 5 (TGACGTCA)
Both vectors were transfected in a stable way in CHO-K1 cells and positive clones were selected on the basis of the best Luciferase induction obtained upon 4 hrs. incubation with the NO donor compound SNAP (Fig. 1).

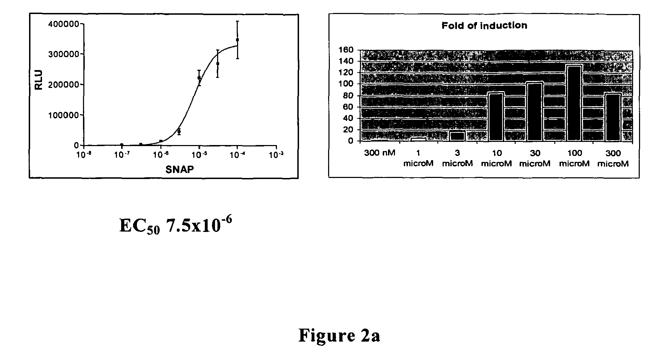

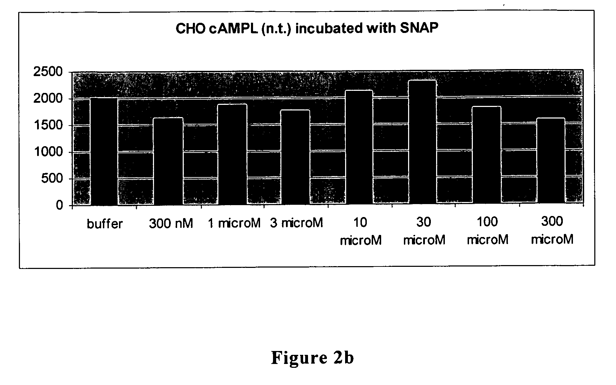

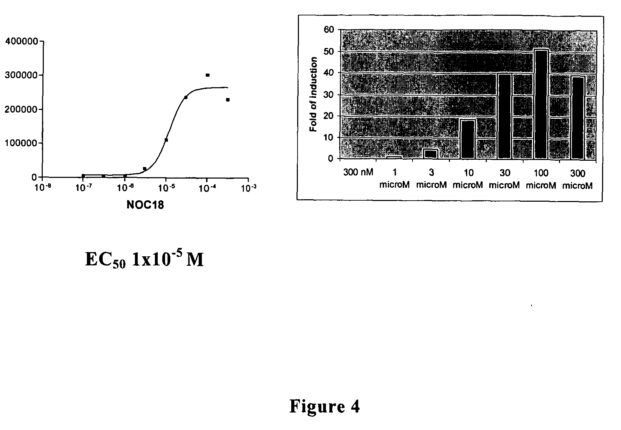

The most responsive clone coming from 3 rounds of limiting dilutions was used to test the activity of different compounds such as NO-donors and NO-independent activators, in the presence or absence of the known sGC inhibitor ODQ (Fig. 2-4).

The results are illustrated in the attached Figures, where:

### Figure 1: 3rd LD clone re-test

12 best clones coming from 3rd LD were incubated in 50 µl serum free medium with SNAP for 4h: luciferase activity was measured after injection of 50 µl Bright-GloTM (Promega) at the CCD camera (60" measurement, high sensitivity).

Experimental conditions: 5000 c/w in 96 wp format, 48 h after seeding.

Clone 7 was chosen as final clone for further characterization.

### Figure 2: (a) EC₅₀ calculation for the NO-donor SNAP

5000 c/w were seeded in 96 wp. 48 h after seeding the cells were incubated 4 h with 50 µl of SNAP in serum free medium (SFM). (**b**) The experiment was performed in parallel also on not transfected cells.

Luciferase activity was detected after injection of 50 µl Bright-GloTM (Promega) at the CCD camera (60" measurement, high sensitivity). Published EC₅₀ 1,7x10⁻⁶M.

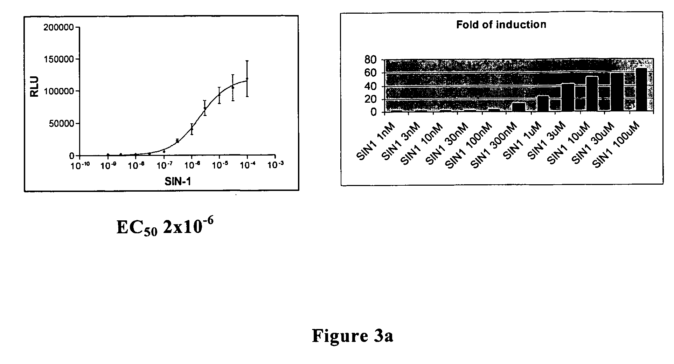

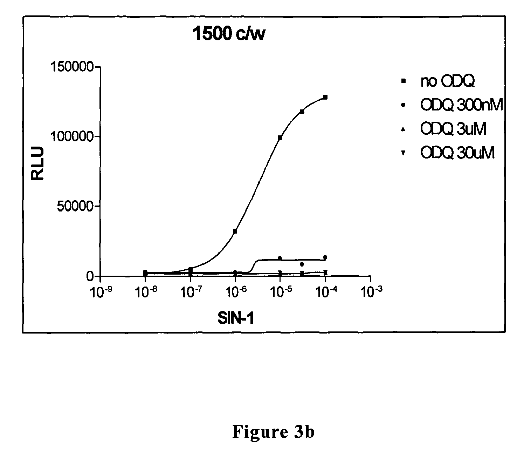

### Figure 3: (a) EC₅₀ calculation for the NO-donor SIN-1

1500 c/w were seeded in 384 wp. 48 h after seeding the cells were incubated 4 h with SIN-1 in Tyrode buffer (25 µl). Luciferase activity was detected after injection of 25 µl of Bright-GloTM (Promega) at the CCD camera (60" measurement, high sensitivity). Published EC₅₀ 10⁻⁷-10⁻⁶M.

### (b) Inhibition of SIN-1 activity by ODQ

1500 c/w were seeded in 384 wp; 48 h after seeding a dose response of SIN in the absence or presence of ODQ (300 nM, 3 µM, 30 µM) was performed. Total volume of incubation 25 µl in Tyrode buffer.

### Figure 4: EC₅₀ calculation for the NO-donor NOC-18.

5000 c/w were seeded in 96 wp. 48 h after seeding the cells were incubated 4 h with 50 µl of NOC18 in serum free medium (SFM). Luciferase activity was detected after injection of 50 µl Bright-GloTM (Promega) at the CCD camera (60" measurement, high sensitivity).

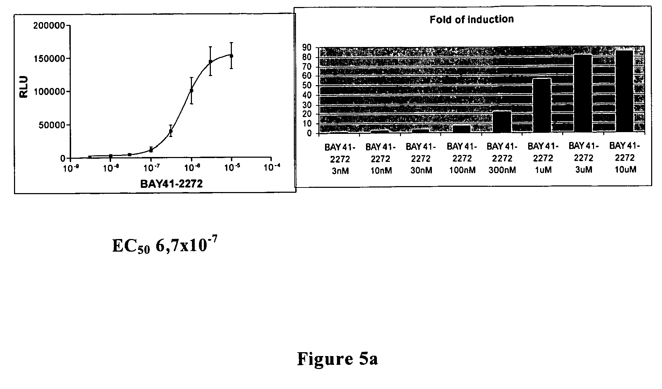

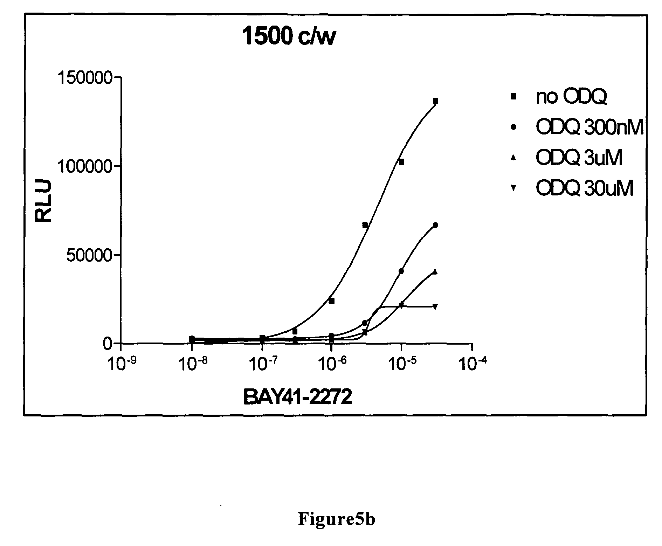

### Figure 5: (a) EC₅₀ calculation for the sGC stimulator BAY41-2272

1500 c/w were seeded in 384 wp. 48 h after seeding the cells were incubated 4 h with BAY41-2272 in Tyrode buffer 25 µl. Luciferase activity was detected after injection of 25ul Bright-GloTM (Promega) at the CCD camera (60" measurement, high sensitivity). Published EC₅₀ 6x10⁻⁷M.

### (b) Inhibition of BAY41-2272 activity by ODQ

1500 c/w were seeded in 384 wp; 48h after seeding a dose response of BAY41-2272 in the absence or presence of ODQ (300 nM, 3 µM, 30 µM) was performed. Total volume of incubation 25 µl in Tyrode buffer. BAY41-2272 stimulating effect on sGC is competed by ODQ.

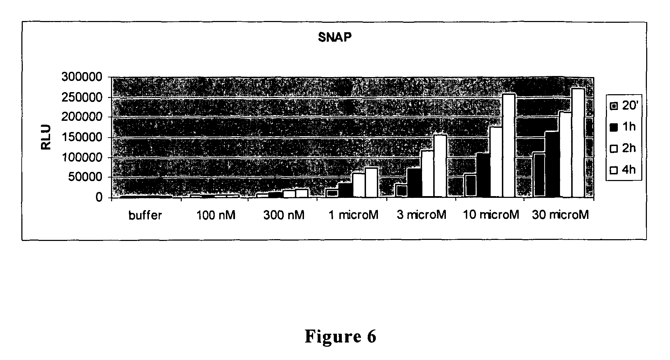

### Figure 6: Kinetic and dose response experiments with SNAP

5000 c/w were seeded in 96 wp. 48 h after seeding the cells were incubated with increasing concentration of SNAP: after times of incubation indicated the compound was replaced with medium serum free. Total incubation time 4 h.

### MATERIALS AND METHODS

### PCR analysis

Rat soluble Guanylate Cyclase (α3 and β1 subunits):
alpha 3 subunit NM_017090; coding region: 690 aa long
beta 1 subunit NM_012769; coding region: 619 aa long
Rat brain cDNA was purchased from Clontech (cat. N° 637312). 2 µl of cDNA was used as template in PCR analysis for non quantitative expression analysis. In addition a negative control was performed with no template.

Standard PCR procedure were as indicated by Perkin Elmer. PCR protocol was as follows: 
Primers:
Primer sGCalpha UP (A): 5'CGGCTAATAAGGAGGAAACCAC3'
Primer sGCalpha LOW (B): 5'AGAAACATGCGGCTCACTAATCTA3'
Primer sGCbeta UP (C): 5'CGGACACCATGTACGGTTTTGTGA3'
Primer sGCbeta LOW (D) 5'GGCTGCCATTCAGTTTTCATCCTG3'
 PCR reaction mix:

| | |
|---|---|
| 2 µl | template |
| 5 µl | 10 x Pfx Buffer (GIBCO-LifeTechnologies) |
| 1.5 µl | 10 mM dNTPs |
| 1 µl | 50 mM MgSO₄ (GIBCO-LifeTechnologies) |
| 2.5 µl | primer A (10 µM) |
| 2.5 µl | primer B (10 µM) |
| 2.5 U | Platinum Pfx (GIBCO-LifeTechnologies) |
| 35 µl | H₂O |

Amplification protocol performed in Perkin Elmer 9700 thermocycler:
1 time the following step:

| | |
|---|---|
| pre PCR | 2' at 94°C |

40 times the following steps:

| | |
|---|---|
| denaturation | 30" at 94°C |
| annealing | 1' at 56°C |
| elongation | 2' 10" at 68°C |

1 time the following step:

| | |
|---|---|
| elongation | 7' at 68°C |

Expected length of specific PCR product: 1309 bp (690 bp for A/B amplification; 619 bp for C/D).

Amplification products were analysed by electrophoresis on 1% agarose gel in 1xTAE running buffer following standard procedure, as described by Maniatis et al.

### Site Directed Mutagenesis

Mutations into the β subunit of sGC were inserted by PCR using the wild type gene as template, in particular primers sGCbeta UP and sGCbetaE473K LOW were used to amplify the 5' portion of the gene, primers sGCbetaE473K UP and sGCbetaC541D LOW were used to amplify a central fragment, primers sGCbetaC541D UP and sGCbeta LOW were used to amplify the 3' portion of the gene.

To reconstitute the full length mutated gene, these three discrete fragments were used as template in a final PCR reaction performed with primers sGCbeta UP sGCbeta LOW.

Amplification protocol performed in Perkin Elmer 9700 thermocycler:
1 time the following step:

| | |
|---|---|
| pre PCR | 2' at 94°C |

20 times the following steps:

| | |
|---|---|
| denaturation | 30" at 94°C |
| annealing | 1' at 52°C |
| elongation | 30" at 68°C |

1 time the following step:

| | |
|---|---|
| elongation | 7' at 68°C |

The mutation R592Q into the α subunit of sGC was inserted by site directed mutagenesis (QuikChange XL Site-Directed Mutagenesis Kit from Stratagene), using primers sGCalphaR592Q UP and sGCalphaR592Q LOW.

Amplification protocol performed in Perkin Elmer 9700 thermocycler:
1 time the following step:

| | |
|---|---|
| pre PCR | 1' at 95°C |

18 times the following steps:

| | |
|---|---|
| denaturation | 30" at 95°C |
| annealing | 1' at 55°C |
| elongation | 13' at 68°C |

1 time the following step:

| | |
|---|---|
| elongation | 7' at 68°C |

PCR reaction mix:

| | |
|---|---|
| 1 µl | template |
| 5 µl | 10 x Turbo Pfu Buffer (Stratagene) |
| 1 µl | 20mM dNTPs |
| 2.5 µl | primer A (10 µM) |
| 2.5 µl | primer B (10 µM) |
| 1 µl | 2.5 U/µl Turbo Pfu (Stratagene) |
| 37 µl | H₂O |

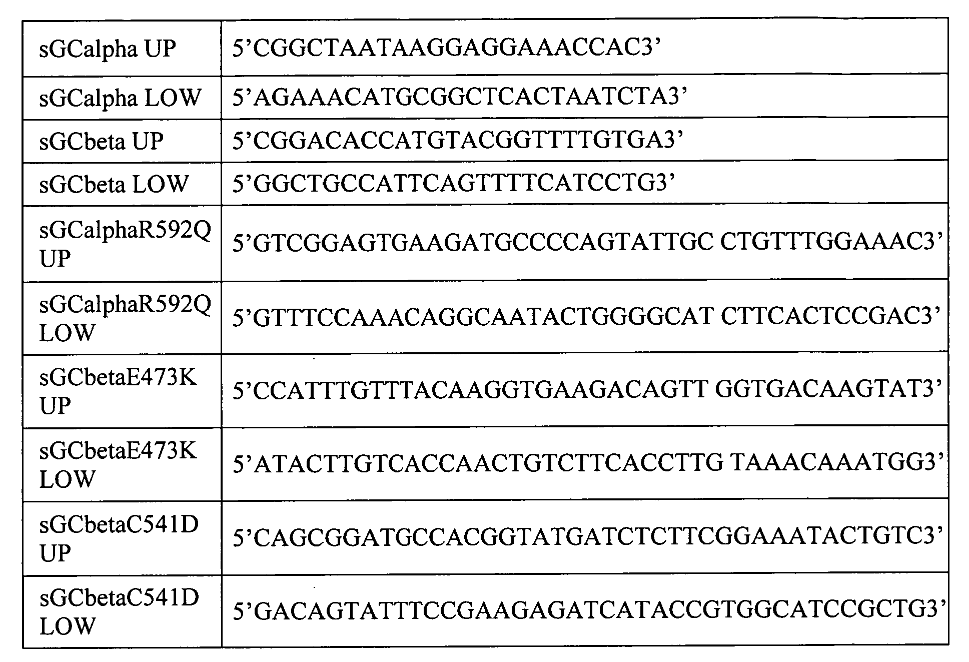

### Cloning procedure:

### pIRESsGCβαmut

Each of the two mutated subunits of sGC was cloned into a bi-cistronic mammalian expression vector (pIRES, purchased from Clontech) that allows the simultaneous translation of two consecutive ORF (Open Reading Frame) from the same messenger RNA, through an Internal Ribosome Entry Site (IRES) element, thus generating the so-called pIRESsGCβα vector.

The mutated β subunit of sGC described above was cloned into EcoRI unique restriction site of pIRES.

The pIRESsGCβ vector was subsequently digested with Smal to insert the mutated α subunit of sGC downstream the IRES element.

All the constructs obtained were verified by full-length dideoxy sequencing.

### Cell culture:

Culture medium, seeding and incubation: DMEM/F 12 with Glutamax (GIBCO cod.31331-028), 10% FBS, 1% Pen./Strep. (Invitrogen cod.15140-122), 25 mM Hepes Buffer Solution (GIBCO cod.15630-056), 1.0 mM Sodium Pyruvate (GIBCO cod.11360-039), 1.5 g/L Sodium Bicarbonate (GIBCO cod. 25080-060), 0.5 mg/ml G418 (Calbiochem cod.345812).

Preculture conditions: Cells were seeded for experiments when 70-80% confluent.

Cell culture conditions: Split twice a week: 3.0x10⁵ cells/flask T75 (recovery: 8x10⁶ cells).

### Clone Selection Process:

CHO cAMPL, previously generated by Axxam, were transfected with pIRESsGCβαmut.

After eight days of G418 selection, the stable transfected cells were put in 1st limiting dilution (1st LD) at 1 c/w in 96 wp format.

After two weeks, plates were incubated 4h with SNAP 100 µM and measured at CCD camera after injection of Bright-GloTM (Promega). The 9 best responding clones were re-tested in a dose response experiment as counted cells.

The 3 best responding clones were put in 2nd LD (0.3 c/w, 96wp).

After two weeks, plates were replicated and after 48 h incubated 4 h with SNAP 100 µM and read at CCD camera after injection of Bright-GloTM (Promega). The best responding 12 clones were re-tested in a dose response experiment as counted cells.

The four best responding clones were put in 3rd LD (0.3 c/w, 96 wp format).

The final clone was chosen after the 3rd LD selected with SNAP 10 µM.

Complete optimization of the assay was performed on the best responding clone 7.

### Reference Compounds:

**SNAP** (Tocris cat# 0598) was dissolved in DMSO at a concentration of 100 mM and stored in aliquots at -20°C. Working solution was freshly prepared in Serum Free Medium.

**SIN** (Tocris cat# 0 756) was dissolved in water at a concentration of 100 mM and stored in aliquots at -20°C.

**NOC18** (Calbiochem cat# 487957) was dissolved in water at a concentration of 100 mM and stored in aliquots at -20°C.

**ODQ** (Calbiochem cat# 495320) was dissolved in DMSO at a concentration of 100 mM and stored in aliquots at -20°C.

**BAY41-2272** was purchased from Alexis, dissolved in DMSO at a concentration of 10 mM and stored in aliquots at -20°C.

Working solutions for SIN-1 and BAY41-2272 and BAY58-2667 were freshly prepared in standard Tyrode buffer.

**Tyrode Buffer composition:** NaCl 130 mM, KCl 5 mM, CaCl₂ 2 mM, MgCl₂ 1 mM, NaHCO₃ 5 mM e HEPES 20 mM pH 7.4.

**Bright-Glo™:** Promega cat#E2620.

### REFERENCES

Garbers, D.L., Koesling, D., and Schultz, G. (1994) *Mol. Biol. Cell* 5, 1-5.
Dessauer, C.W., Scully, T.T., and Gilman, A.G. (1997) *J. Biol. Chem.* 272, 22272-22277.
Fink JS, Verhave M, Kasper S, Tsukada T, Mandel G, Goodman RH., Proc Natl Acad Sci U S A. 1988 Sep;85(18):6662-6.
Fitzgerald, LR, Manan, IJ, Dytko, GM, Wu, HL, Nambi,P, (1999) Analytical Biochemistry, 275, 54-61.
Liu, Y., Ruoho, A.E., Rao, V.D., and Hurley, J.H. (1997) *Proc. Natl. Acad. Sci. U.S.A.* 94, 13414-13419.
Ray A, Sassone-Corsi P, Sehgal PB. Mol Cell Biol. 1989 Dec; 9(12):5537-47.
Schmidt P.M., Schramm M., Schroeder H, Wunder F. and Stasch J.P., J Biol. Chem., 2004, vol. 279, p. 3025-3032.
Stasch J.P., Nature, 2001, vol. 410, p. 212-215.
Sunahara, R.K., Dessauer, C.W., and Gilman, A.G. (1996) *Annu. Rev. Pharm. Toxicol.* 36, 461-480.
Sunahara, R.K., Dessauer, C.W., Whisnant, R.E., Kleuss, C., and Gilman, A.G. (1997) *J. Biol. Chem.* 272, 22265-22271.
Sunahara, R.K., Beuve, A., Tesmer, J.J.G., Sprang, S.R., Garbers, D.L., and Gilman, A.G., J Biol. Chem., 1998, vol. 237, p. 16332-16338.
Tang, W.-J., and Gilman, A.G. (1995) *Science* 268, 1769-1772.
Tesmer, J.J.G., Sunahara, R.K., Gilman, A.G., and Sprang, S.R. (1997) *Science* 278, 1907-1916.

## Claims

1. A method for determining the soluble guanylate cyclase (sGC) enzymatic activity in a cultured eukaryotic or prokaryotic cell, which comprises:
A) introducing into the cell:
i) a vector encoding a reporter gene functionally linked to a CRE or MRE-CRE inducible promoter,
and, simultaneously or separately,
ii) a vector encoding a soluble guanylate cyclase able to catalyze the conversion of ATP into cyclic AMP;
B) contacting the cell with a compound that stimulates or inhibits sGC enzymatic activity;
C) determining the activity of the reporter gene.

2. A method of identifying molecules that modulate sGC activity in a cultured eukaryotic or prokaryotic cell, which comprises:
A) introducing into the cell:
i) a vector encoding a reporter gene functionally linked to a CRE or MRE-CRE inducible promoter,
and, simultaneously or separately,
ii) a vector encoding a soluble guanylate cyclase able to catalyze the conversion of ATP into cyclic AMP;
B) contacting the cell with a candidate molecule;
C) determining the activity of the reporter gene.

3. A method of modulating the expression of a target gene in a cultured eukaryotic or prokaryotic cell, which comprises:
A) introducing into the cell:
i) a vector encoding the target gene functionally linked to a CRE or MRE-CRE inducible promoter,
and, simultaneously or separately,
ii) a vector encoding a soluble guanylate cyclase able to catalyze the conversion of ATP into cyclic AMP;
B) contacting the cell with a molecule able to stimulate sGC enzymatic activity in a NO-dependent or NO-independent manner;

4. A method according to claims 1-3, wherein the MRE-CRE inducible promoter contains 3 MRE and 5 CRE elements.

5. A method according to claims 1-3, wherein a vector encoding rat soluble guanylate cyclase is used.

6. A method according to claim 5, wherein said rat sGC carries Arg592→Gln mutation on the α subunit and Glu473→Lys and Cys541→Asp mutations on the β subunit.

7. A method according to claims 1-2, wherein the reporter gene codes for a protein selected from luciferase, Green Fluorescent Protein (GFP) and a Photoprotein.

8. A method according to claim 2, wherein the candidate molecule is able to stimulate or inhibit sGC activity in a NO-dependent or NO-independent modality.

9. A method according to claim 3, wherein the target gene is selected from ion channels, GPCRs and Nuclear Hormone Receptors.

10. A prokaryotic or eukaryotic host cell containing i) a vector encoding a reporter gene functionally linked to a CRE or MRE-CRE inducible promoter and ii) a vector encoding a soluble guanylate cyclase able to catalyze the conversion of ATP into cyclic AMP.

11. A eukaryotic host cell according to claim 8, which is a CHO cell.

12. The use of a soluble guanylate cyclase able to catalyze the conversion of ATP into cyclic AMP for the screening of compounds that modulate sGC enzymatic activity.

13. The use of a host cell according to claims 10-11 as a tool for detection of intracellular NO-release.


SEQUENCE LISTING

<110> AXXAM S.r.l.

<120> METHODS AND ASSAYS FOR DETECTING GUANYLATE CYCLASE ACTIVITY

<130> SCB1387EUR

<160> 11

<170> PatentIn version 3.1

<210> 1
<211> 32
<212> DNA
<213> Homo sapiens

<400> 1
atgctaaagg acgtcacatt gcacaatctt aa 32

<210> 2
<211> 22
<212> DNA
<213> Rattus sp.

<400> 2
cggctaataa ggaggaaacc ac 22

<210> 3
<211> 24
<212> DNA
<213> Rattus sp.

<400> 3
agaaacatgc ggctcactaa tcta 24

<210> 4
<211> 24
<212> DNA
<213> Rattus sp.

<400> 4
cggacaccat gtacggtttt gtga 24

<210> 5
<211> 24
<212> DNA
<213> Rattus sp.

<400> 5
ggctgccatt cagttttcat cctg 24

<210> 6
<211> 39
<212> DNA
<213> Rattus sp.

<400> 6

gtcggagtga agatgcccca gtattgcctg tttggaaac                                39

<210>  7
<211>  39
<212>  DNA
<213>  Rattus sp.

<400>  7
gtttccaaac aggcaatact ggggcatctt cactccgac                                39

<210>  8
<211>  39
<212>  DNA
<213>  Rattus sp.

<400>  8
ccatttgttt acaaggtgaa gacagttggt gacaagtat                                39

<210>  9
<211>  39
<212>  DNA
<213>  Rattus sp.

<400>  9
atacttgtca ccaactgtct tcaccttgta aacaaatgg                                39

<210>  10
<211>  39
<212>  DNA
<213>  Rattus sp.

<400>  10
cagcggatgc cacggtatga tctcttcgga aatactgtc                                39

<210>  11
<211>  39
<212>  DNA
<213>  Rattus sp.

<400>  11
gacagtattt ccgaagagat cataccgtgg catccgctg                                39